# EUROPEAN PATENT APPLICATION

(11) **EP 4 643 790 A1**
(43) Date of publication of application: **05.11.2025**
(21) Application number: 25172977.8
(22) Date of filing: 28.04.2025
(51) Int. Cl.: A61B 17/04, A61B 17/34

(54) **ANCHOR AND ANCHORING DEVICE**

(30) Priority: 30.04.2024 CN 202410544027
(71) Applicant: Micro-Tech (Nanjing) Co., Ltd., Nanjing, Jiangsu 210032 (CN); Shengjing Hospital of China Medical University, Shenyang, Liaoning 110004 (CN)
(72) Inventor: SUN, Siyu, Shenyang City, Liaoning, 110004 (CN); JIN, Hongyan, Nanjing, Jiangsu, 210032 (CN); HU, Jie, Nanjing, Jiangsu, 210032 (CN); MA, Xiaojun, Nanjing, Jiangsu, 210032 (CN); WEI, Jianyu, Nanjing, Jiangsu, 210032 (CN); CHENG, Xue, Nanjing, Jiangsu, 210032 (CN)
(74) Representative: Giopato, Paolo

(57) **Abstract**

Provided are an anchor and an anchoring device, including: a traction line, which is a flexible member; an anchoring body connected to a distal end of the traction line and can be pulled by the traction line; a driving tube, which is at least sleeved on at least a portion of the traction line and is configured to drive the anchoring body to move along the puncture needle so that the anchoring body enters the target tissue; a recovery line, which is connected to the anchoring body, wherein the recovery line is configured to pull the anchoring body so that the anchoring body is separated from the target tissue along the puncture channel of the target tissue, and can reduce the friction between the anchoring body and the tissue during the process of the recovery line pulling the anchoring body to retrieve it, thereby alleviating or avoiding damage to the tissue.

## Description

### TECHNICAL FIELD

The present invention relates to the technical field of medical devices, and in particular to an anchor and an anchoring device.

### BACKGROUND ART

Endoscopic Ultrsonography (EUS for short) is a minimally invasive surgery for evaluating digestive tract and lung diseases. It involves medical surgeries on the patient's gastrointestinal tract. For example, in gallbladder drainage surgery guided by endoscopic ultrsonography, a fixator needs to be implanted between the gallbladder and the intestine. The fixator is used as a fistula to achieve communication between the gallbladder and the intestine.

In order to ensure the stability of the fixator, an anchor is usually implanted into the gallbladder through the intestine under the guidance of an endoscope. By pulling the traction line of the anchor outward, the anchoring body of the anchor drives the gallbladder to tightly adhere to the side wall of the intestine to achieve the anchoring of the gallbladder. In addition, a recovery line is connected to the anchoring body. When the operation is completed, the recovery line can be pulled to remove the anchoring body from the body.

However, during the process of pulling the anchoring body with the recovery line to retrieve, the friction between the anchoring body and the wall of the tissue channel such as the gallbladder is large due to the resilience force between the anchoring body and the traction line, which causes damage to the tissue.

### SUMMARY

The embodiment of the present invention provides an anchoring device and an anchor, which can reduce the friction between the anchoring body and the tissue during the process of the recovery line pulling the anchoring body to retrieve, thereby alleviating or avoiding damage to the tissue.

On the one hand, the embodiment of the present invention provides an anchor, including:
a traction line, which is a flexible member;
an anchoring body, connected to the distal end of the traction line, and capable of being pulled by the traction line;
a driving tube, at least sleeved on at least part of the traction line, and configured to drive the anchoring body to move along the puncture needle so that the anchoring body enters the target tissue; and
a recovery line, which is connected to the anchoring body, wherein the recovery line is configured to pull the anchoring body so that the anchoring body is separated from the target tissue along a puncture channel of the target tissue; and the puncture channel is a channel on the target tissue formed by puncturing the target tissue by the puncture needle.

In one embodiment, the driving tube is sleeved on the recovery line, and the anchoring body is located outside the distal end of the driving tube; and
when the anchoring body is located in the puncture needle, a proximal end of the anchoring body abuts the distal end of the driving tube, so that when the anchoring body extends out of the puncture needle, the anchoring body can be flipped under pushing of the driving tube and pulling of the traction line.

In one embodiment, a limiting groove and a side notch communicated with the limiting groove are formed on the anchoring body, the limiting groove and the side notch both extend along a length direction of the anchoring body, and one ends of the limiting groove and the side notch both run through a proximal end of the anchoring body,
wherein when the proximal end of the anchoring body is located at the puncture needle, at least part of the traction line is located in the limiting groove, and during a process of the traction line pulling the anchoring body to flip, a part of the traction line detaches from the anchoring body through the side notch.

In one embodiment, an avoidance opening is formed at one end of the limiting groove located at the proximal end of the anchoring body, one end of the avoidance opening extends to the side notch, and the other end of the avoidance opening extends to a side of an axis of the limiting groove away from the side notch.

In one embodiment, a gap is provided between the other end of the avoidance opening and a groove bottom of the limiting groove,
wherein the groove bottom of the limiting groove is arranged opposite to the side notch.

In one embodiment, when the proximal end of the anchoring body is located at the puncture needle, the traction line extends along the axis of the limiting groove; or
when the proximal end of the anchoring body is located at the puncture needle, the traction line is located between the axis of the limiting groove and the side notch.

In one embodiment, when the anchoring body is flipped to the anchoring position, the position where the traction line is detached from the anchoring body is located at the center position of the anchoring body along a length direction.

In one embodiment, one end of the recovery line is located at the center position of the anchoring body along the length direction; or
one end of the recovery line is located between the center position of the anchoring body along the length direction and the distal end of the anchoring body,
wherein the distal end of the traction line is located between the center position and the distal end of the anchoring body, and the traction line extends toward the proximal end of the anchoring body; the recovery line extends toward the distal end of the anchoring body, and extends out of the anchoring body from the distal end of the anchoring body; and the recovery line is configured to drive the distal end of the anchoring body to flip toward the puncture channel.

In one embodiment, a line placement cavity is formed on the anchoring body,
wherein the line placement cavity communicates with a groove cavity of the limiting groove through a penetration hole, a distal end of the traction line is limited in the line placement cavity, and the traction line extends from the penetration hole into the limiting groove.

In one embodiment, a first limiting portion is formed at the distal end of the traction line, and the first limiting portion is embedded in the line placement cavity so that the distal end of the traction line is limited in the line placement cavity.

In one embodiment, the anchoring body also forms a limiting channel, the limiting channel communicates with the line placement cavity of the anchoring body, and one end of the limiting channel runs through the distal end of the anchoring body; and
one end of the recovery line is fixed in the line placement cavity, and the recovery line extends out of the distal end of the anchoring body through the limiting channel.

In one embodiment, a second limiting portion is formed at one end of the recovery line, and the second limiting portion is clamped in the line placement cavity so that one end of the recovery line is limited in the line placement cavity.

In one embodiment, one end of the recovery line is connected to a first limiting portion at the distal end of the traction line, and the first limiting portion is embedded in the line placement cavity so that one end of the recovery line is limited in the line placement cavity.

In one embodiment, a side wall of the anchoring body is recessed inwardly to form a line placement groove, and a groove cavity of the line placement groove is configured as the line placement cavity; and
the limiting channel is a limiting hole formed on the anchoring body, and the two ends of the limiting hole respectively run through the line placement groove and the distal end of the anchoring body.

In one embodiment, a mounting channel is formed in the anchoring body, one end of the mounting channel communicates with the penetration hole, and the other end of the mounting channel runs to a distal end of the anchoring body,
wherein the distal end of the anchoring body has an end cap, the end cap is embedded in the mounting channel from the distal end of the anchoring body, and one end is spaced from the penetration hole; and
the line placement cavity is formed between one end of the end cap and the penetration hole, the inner cavity of the end cap forms the limiting channel, and one end of the end cap has a first through hole to communicate the line placement cavity with the limiting channel, and the other end of the end cap has a second through hole to communicate the limiting channel with the outside of the anchoring body.

In one embodiment, at least a part of the recovery line is located in the driving tube so as to enter the target tissue under the drive of the driving tube.

On the other hand, the embodiment of the present invention also provides an anchoring device, including:
a puncture needle, wherein a distal end of the puncture needle is configured to extend into a target tissue;
the anchor as described above; and
the anchoring body of the anchor moves along the puncture needle under the drive of the driving tube to enter the target tissue.

The embodiment of the present invention provides an anchoring device and an anchor, the traction line is arranged as a flexible member, and the traction line is disposed in a driving tube, so that when the anchoring body at one end of the traction line needs to be implanted, the puncture needle can be implanted into the target tissue first, and then the traction line and the anchoring body are driven by the driving tube to move along the puncture needle toward the target tissue. When the anchoring body extends out of the distal end of the puncture needle, the anchoring body can be flipped to have an angle with the traction line, so that the anchoring body can be stopped at the inner wall of the target tissue, and the traction line can be pulled so that the target tissue is closely attached to other tissues under the drive of the anchoring body. In addition, by arranging the traction line as a flexible member, it is possible to avoid a resilience force between the flexible member and the anchoring body. In this way, when the recovery line drives the anchoring body to be recovered, the anchoring body will not rebound outward when retreating along the puncture channel, thereby reducing the friction between the anchoring body and the inner wall of the puncture channel, thereby reducing or avoiding damage to the tissue (target tissue and first tissue).

### BRIEF DESCRIPTION OF DRAWINGS

Figure 1 is a schematic diagram of an assembly of a puncture needle and a traction line provided in an embodiment of the present invention;
Figure 2 is a structural schematic diagram of an anchor provided in an embodiment of the present invention in a first state;
Figure 3 is a structural schematic diagram of an anchor provided in an embodiment of the present invention in a second state;
Figure 4a is a structural schematic diagram of an anchor provided in an embodiment of the present invention in a first recovery state;
Figure 4b is a structural schematic diagram of an anchor provided in an embodiment of the present invention in a second recovery state;
Figure 4c is a structural schematic diagram of an anchor provided in an embodiment of the present invention in a third recovery state;
Figure 4d is a structural schematic diagram of an anchor provided in an embodiment of the present invention in a fourth recovery state;
Figure 5 is a structural schematic diagram of one of the anchors provided in an embodiment of the present invention;
Figure 6 is a sectional view of the assembly of the anchor, traction line and recovery line in Figure 5;
Figure 7 is a sectional view of the anchor in Figure 6;
Figure 8 is a partial enlarged view of part A in Figure 2;
Figure 9 is a structural schematic diagram of the end cap in Figure 8;
Figure 10 is a structural schematic diagram of another anchor provided in an embodiment of the present invention; and
Figure 11 is a partial structural schematic diagram of an anchor provided in an embodiment of the present invention.

Explanation of reference numerals:
10-anchor; 20-puncture needle; 30-first tissue; 40-target tissue;
100-traction line; 200-anchoring body; 300-driving tube; 400-recovery line;
110-first limiting portion; 210-limiting groove; 220-line placement cavity; 220a-line placement groove; 230-limiting channel; 230a-limiting hole; 240-avoidance opening; 250-end cap; 260-mounting channel; 410-second limiting portion;
210a-groove bottom; 210b-side notch; P-flip fulcrum; 210c-penetration hole; 250a-first through hole; 250b-second through hole.

### DETAILED DESCRIPTION OF EMBODIMENTS

In order to enable those skilled in the art to better understand the technical solutions in the present invention, the technical solutions in the embodiments of the present invention will be clearly and completely described below in conjunction with the drawings in the embodiments of the present invention. Obviously, the described embodiments are only part of the embodiments of the present invention, not all of the embodiments. Based on the embodiments in the present invention, all other embodiments obtained by those ordinary skilled in the art without creative work should fall within the scope of protection of the present invention.

It should be noted that many specific details are described in the following description to facilitate a full understanding of the present invention. However, the present invention can also be implemented in other ways different from those described herein. Therefore, the scope of protection of the present invention is not limited by the specific implementations disclosed below.

In the description of the present invention, it should be understood that the terms "above", "below", "horizontal", "bottom", "inner", "outer" and the like indicate the orientation or position relationship based on the orientation or position relationship shown in the drawings, which is only for the convenience of describing the present invention and simplifying the description, rather than indicating or implying that the device or element referred to must have a specific orientation, be constructed and operated in a specific orientation, and therefore cannot be understood as a limitation on the present invention. In this disclosure, unless otherwise clearly specified and limited, the first feature "above" or "below" the second feature may be the first and second features in direct contact, or the first and second features in indirect contact through an intermediate medium.

In this disclosure, unless otherwise clearly specified and limited, the terms "connected with", "connected to", "fixed to" and the like should be understood in a broad sense, for example, it can be a fixed connection, a detachable connection, or an integral body; it can be directly connected, or indirectly connected through an intermediate medium, it can be the internal connection of the two elements or the interaction relationship between the two elements. However, the direct connection indicates that the two connected bodies do not establish a connection relationship through a transition structure, but are only connected to form a whole through a connecting structure. For those ordinary skilled in the art, the specific meanings of the above terms in this disclosure can be understood according to the specific circumstances.

In this disclosure, the descriptions involving "first", "second", etc. are only for descriptive purposes, and cannot be understood as indicating or implying their relative importance or implicitly indicating the number of technical features indicated. Therefore, the features defined as "first" and "second" can explicitly or implicitly include at least one of the features.

Endoscopic Ultrsonography (EUS) is a minimally invasive surgery for evaluating digestive tract and lung diseases. In medical surgeries involving the gastrointestinal tract of patients, it is necessary to implant a fixator between the two drainage tissues, and use the fixator as a fistula to achieve the connection between the two tissues. For example, in gallbladder drainage surgery guided by endoscopic ultrasound, it is necessary to implant a fixator between the gallbladder and the intestine, and use the fixator as a fistula to achieve the connection between the gallbladder and the intestine.

It is understandable that because the gallbladder and the intestine are in a free state, the process of installing the fixator becomes more difficult.

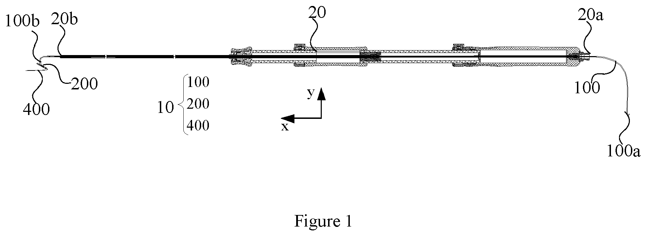

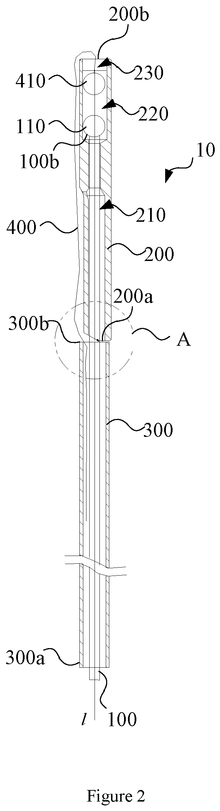

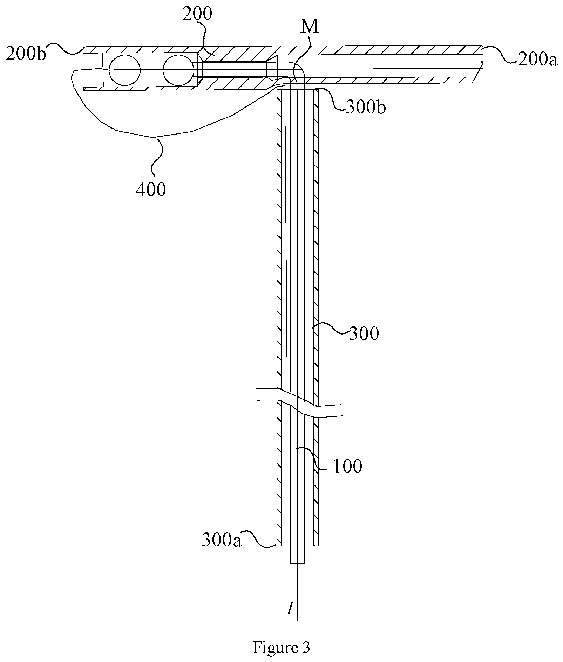

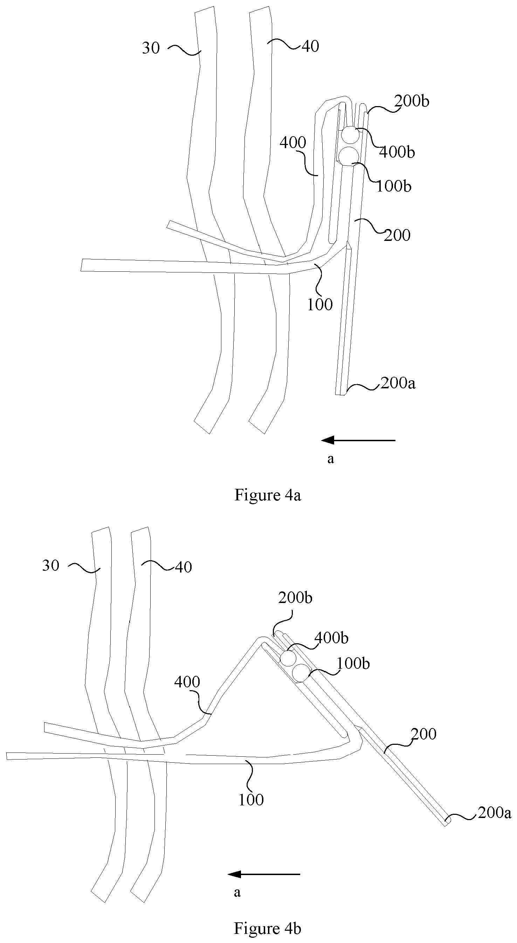

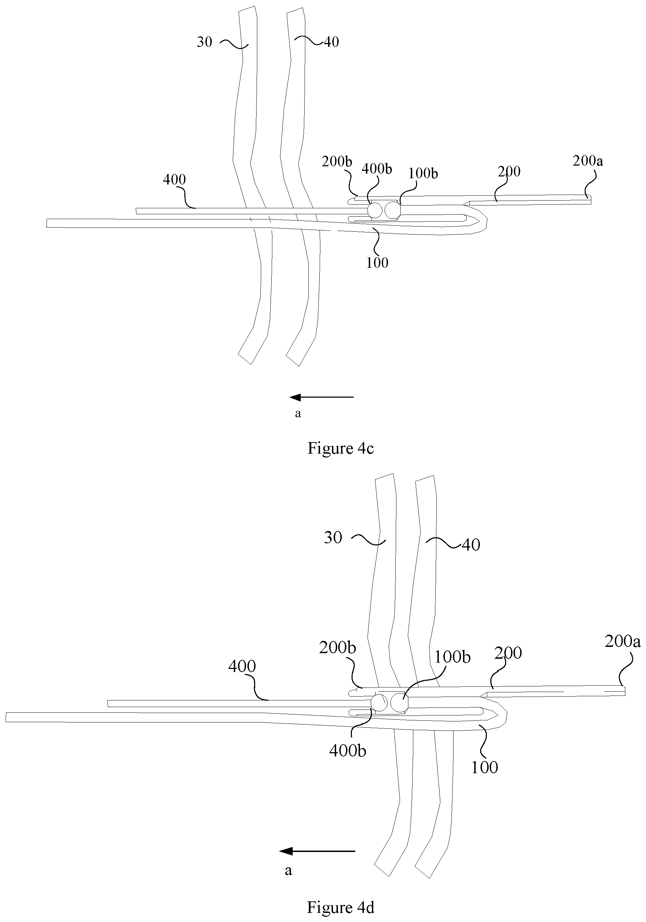

In order to facilitate the installation of the fixator, an embodiment of the present invention provides an endoscopic ultrasound anchoring system, including an endoscope and an anchoring device. Figure 1 is a schematic diagram of the assembly of the puncture needle and the traction line provided in an embodiment of the present invention; Figure 2 is a structure schematic diagram of the anchor provided in an embodiment of the present invention in the first state; Figure 3 is a structure schematic diagram of the anchor provided in an embodiment of the present invention in the second state; Figure 4a is a structure schematic diagram of the first recovery state of the anchor provided in an embodiment of the present invention; Figure 4b is a structure schematic diagram of the second recovery state of the anchor provided in an embodiment of the present invention; Figure 4c is a structure schematic diagram of the third recovery state of the anchor provided in an embodiment of the present invention; Figure 4d is a structure schematic diagram of the fourth recovery state of the anchor provided in an embodiment of the present invention. Referring to Figure 1 to Figure 3, the anchoring device includes an anchor 10, and the anchor 10 has a traction line 100 and an anchoring body 200. The anchoring body 200 is connected to the distal end of the traction line 100 (refer to 100b in Figure 1).

When surgery is required, the anchoring body 200 can be implanted into the target tissue 40 such as the gallbladder through the first tissue 30 such as the intestine under the guidance of the endoscope. For example, the cannula of the endoscope can be introduced into the intestine first, and then the anchor 10 can be placed along instrument channel of the cannula. When the anchoring body 200 of the anchor 10 reaches the gallbladder through the intestine, the traction line 100 is pulled outward so that the anchoring body 200 stops on the inner wall of the target tissue 40, and the traction line 100 is continued to be pulled so that the anchoring body 200 drives the gallbladder to stick close to the side wall of the intestine, that is, the gallbladder is anchored, so that the gallbladder is firmly fixed to the outside of the intestine. On the one hand, it is convenient to install a fixator between the intestine and the gallbladder, and on the other hand, it also makes the fixator more stable between the intestine and the gallbladder.

Continuing to refer to Figure 1, the anchoring device may also include a puncture needle 20. When the anchor 10 needs to be implanted, the puncture needle 20 may be implanted through the intestine and gallbladder in sequence under the guidance of an endoscope. When the distal end of the puncture needle 20, i.e., the needle tip, reaches the preset position in the gallbladder, the anchor 10 is moved along the needle channel of the puncture needle 20 until the anchoring body 200 of the anchor 10 extends out of the distal end of the puncture needle 20, thereby achieving the purpose of implanting the anchoring body 200 into the gallbladder.

In order to facilitate the implantation of the anchor 10, in the related art, the traction line 100 is formed of a hard material, and there is a resilience force between the traction line 100 and the anchoring body 200, i.e., the anchoring body 200 has a force to rebound toward the anchoring position, wherein the anchoring position is the position when the anchoring body 200 has a preset angle with the traction line 100. During the implantation process, the anchoring body 200 moves along the needle channel of the puncture needle 20 under the push of the hard traction line 100 until the anchoring body 200 extends out of the needle head of the puncture needle 20. The anchoring body 200 then will have a preset angle with the traction line 100 under the action of the resilience force, thereby pulling the traction line 100, so that the anchoring body 200 can drive the gallbladder to stick close to the intestine.

Referring to Figures 4a to 4d, the anchor 10 of the embodiment of the present invention also includes a recovery line 400, which is connected to the anchoring body 200. For example, one end of the recovery line 400 (for example, a fixed end, as shown in 400b in Figures 4a to 4d) is fixed to the anchoring body 200, and the other end of the recovery line 400 (for example, a free end) can extend from the puncture channel of the target tissue 40 and the first tissue 30 into the first tissue 30. The puncture channel is a channel formed by puncturing the target tissue 40 and the first tissue 30 by the puncture needle 20.

After the operation is completed, the grasping forceps can be extended into the first tissue 30 through the instrument channel of the endoscope, and the free end of the recovery line 400 can be grasped under the endoscope, and then withdrawn, so that the anchoring body 200 is moved out of the body through the puncture channel and the instrument channel.

However, during the recovery line 400 pulling the anchoring body 200, the anchoring body 200 always has a resilience force to restore to the anchor position, which makes the friction between the anchoring body 200 and the inner wall of the puncture channel larger when anchoring body 200 passing through the puncture channel, thereby causing damage to tissues such as the target tissue 40 and the first tissue 30.

The embodiment of the present invention provides an anchoring device and an anchor 10, and the traction line 100 is arranged as a flexible member and located in the driving tube 300. In this way, when it is necessary to implant the anchoring body 200 at one end of the traction line 100, the puncture needle 20 can be implanted into the target tissue 40 first, and then the traction line 100 and the anchoring body 200 are driven by the driving tube 300 to move along the puncture needle 20 toward the target tissue 40. When the anchoring body 200 extends out of the distal end of the puncture needle 20, the anchoring body 200 can be flipped to have an angle with the traction line 100, so that the anchoring body 200 can be stopped at the inner wall of the target tissue 40, and the traction line 100 can be pulled so that the target tissue 40 is tightly attached to other tissues under the drive of the anchoring body 200. In addition, the traction line 100 is provided as a flexible member, so that the resilience force between the flexible member and the anchoring body 200 is avoided. In this way, during the recovery line 400 driving the anchoring body 200 to recovery, the anchoring body 200 will not rebound outward when retreating along the puncture channel, thereby reducing the friction between the anchoring body and the inner wall of the puncture channel, and reducing or avoiding damage to the tissue (target tissue 40 and the first tissue 30).

The structure of the anchoring device and the anchor 10 provided in the embodiment of the present invention is described in detail in conjunction with the accompanying drawings.

As shown in Figures 1 to 4, the embodiment of the present invention provides an anchoring device, including an anchor 10 and a puncture needle 20. The distal end of the puncture needle 20 (as shown in 20b in Figure 1) is configured to extend into the target tissue 40. For example, when the anchor 10 needs to be implanted, the puncture needle 20 can be first implanted into the first tissue 30, such as the intestine, along the cannula of the endoscope, and then the puncture needle 20 can be implanted so that the distal end of the puncture needle 20 passes through the tissue arm of the first tissue 30 and the tissue wall of the target tissue 40, and finally reaches the preset position in the target tissue 40, such as the gallbladder. The preset position is a position where the distal end of the puncture needle 20 can be better developed.

Exemplarily, the puncture needle 20 can include but is not limited to a 19G ultrasonic needle.

In the embodiment of the present invention, the anchor 10 includes a traction line 100, and the traction line 100 is a flexible member. For example, the traction line 100 can be a flexible line made of fiber material or other flexible materials. The embodiment of the present invention does not limit the material of the traction line 100, and it only needs to ensure that the traction line 100 is a flexible member.

The anchor 10 includes an anchoring body 200, which is connected to the distal end of the traction line 100 and can be pulled by the traction line 100.

In some examples, the anchoring body 200 can be flipped under the pull of the traction line 100, so that there is an anchoring angle between the anchoring body 200 and the traction line 100.

In other examples, the anchoring body 200 can also be flipped in other ways, for example, the anchoring body 200 can be flipped by a driving member arranged at the distal end of the puncture needle 20 or the traction line 100. The embodiment of the present invention does not limit the flipping way of the anchoring body 200.

The anchoring angle is the angle between the anchoring body 200 and the traction line 100 when the target tissue 40, such as the gallbladder, is pulled stick close to the intestine. Exemplarily, the anchoring angle can be 90° or 90°±10°, which can be adjusted according to the angle of the side wall of the gallbladder so that the anchoring body 200 fits the side wall of the gallbladder. The embodiment of the present invention does not limit the anchoring angle.

The anchor 10 of the embodiment of the present invention includes a driving tube 300, which is at least sleeved on at least a portion of the traction line 100. The anchoring body 200 can move along the puncture needle 20 driven by the driving tube 300. For example, the anchoring body 200 can enter the needle channel of the puncture needle 20 from the proximal end of the puncture needle 20 (refer to 20a in Figure 1) driven by the driving tube 300, and move along the needle channel of the puncture needle 20. When extending out of the distal end of the puncture needle 20, the anchoring body 200 can be flipped under the pull of the traction line 100 to have an angle with the traction line 100, such as an anchoring angle, so that the anchoring body 200 stops at the inner wall of the target tissue 40, thereby the traction line 100 continues to be pulled, so that the target tissue 40 is closely attached to the outer wall of the first tissue 30 under the drive by the anchoring body 200, and the anchoring of the target tissue 40 is completed.

For the convenience of description, the extension direction of the needle channel of the puncture needle 20 can be the x direction, and the radial direction of the needle channel can be the y direction. The radial dimension of the driving tube 300 may be less than or equal to the radial dimension of the puncture needle 20 along the y direction, so that the driving tube 300 can move smoothly along the needle channel of the puncture needle 20.

As shown in Figure 2, in some examples, the driving tube 300 may be sleeved on the first part of the traction line 100. When the anchoring body 200 is located outside the driving tube 300, the first part may be the part between the distal end 100b and the proximal end 100a of the traction line 100, so that a part of the traction line 100 close to the proximal end may be exposed on the proximal end of the driving tube 300, and the operator can pull the traction line 100 from the proximal end of the traction line 100, so that the traction line 100 drives the anchoring body 200 to flip, and the part of the traction line 100 close to the distal end is also located outside the driving tube 300 and can be connected to the anchoring body 200 to achieve the flipping of the anchoring body 200.

When the anchoring body 200 is located inside the driving tube 300, the first part may also be the part between the distal end of the traction line 100 and the position at the first length from the distal end. Exemplarily, the first length may be a suitable length such as 2/3 or 3/4 of the total length of the traction line 100, that is, the proximal end of the traction line 100 (as shown in 100a in Figure 1) and the position at the second length from the proximal end may be exposed on the proximal end of the driving tube 300, so that the operator can pull the traction line 100 from the proximal end of the traction line 100, and the traction line 100 drives the anchoring body 200 to flip.

It can be understood that the second length is equal to the difference between the total length of the traction line 100 and the first length. For example, the second length can be a suitable length such as 1/3 or 1/4 of the total length of the traction line 100. The embodiment of the present invention does not limit the first length and the second length, as long as the driving tube 300 can drive the traction line 100 and the anchoring body 200 to be smoothly implanted into the target tissue along the puncture needle 20, while the operator can pull the proximal end of the traction line 100 to flip the anchoring body 200, and the target tissue can be anchored on the outer wall of the first tissue.

Referring to Figures 1 to 4d, the anchor 10 of the embodiment of the present invention also includes a recovery line 400, which is connected to the anchoring body 200. For example, the fixed end of the recovery line 400 is fixed to the anchoring body 200, and the free end of the recovery line 400 can extend from the puncture channel of the target tissue 40 and the first tissue 30 into the first tissue 30. The puncture channel is a channel on the target tissue 40 and the first tissue 30 for the puncture needle 20 to pass through.

The recovery line 400 is configured to pull the anchoring body 200 so that the anchoring body 200 flips and detaches from the target tissue 40 along the puncture channel of the target tissue 40. For example, after the operation is completed, the grasping forceps can be extended into the first tissue 30 through the instrument channel of the endoscope, and the free end of the recovery line 400 can be grasped under the endoscope, and then withdrawn, so that the anchoring body 200 is moved out of the body through the puncture channel and the instrument channel successively.

It can be understood that the recovery line 400 is a flexible member, and the material of the recovery line 400 and the traction line 100 can be the same or different, as long as the recovery line 400 and the traction line 100 are both flexible members.

Referring to Figures 1 to 4d, the following takes the first tissue 30 as the intestine and the target tissue 40 as the gallbladder as an example to illustrate the implantation process and recovery process of the anchor 10.

After the puncture needle 20 is implanted in place, the traction line 100 and the anchoring body 200 at the distal end of the traction line 100 can be pushed from the proximal end of the puncture needle 20 into the needle channel of the puncture needle 20 through the driving tube 300.

The driving tube 300 is pushed continually so that the anchoring body 200 is pushed toward the distal end of the puncture needle 20 until the anchoring body 200 extends out of the distal end of the puncture needle 20 and extends to the distal end of the driving tube 300, and then the traction line 100 is pulled to flip the anchoring body 200 to the anchoring angle.

It should be noted that in this step, the anchoring body 200 extending out of the distal end of the puncture needle 20 means that the entire part of the anchoring body 200 completely extends out of the distal end of the puncture needle 20, or a part of the anchoring body 200 extends out of the distal end of the puncture needle 20, as long as the traction line 100 is pulled so that the proximal end of the anchoring body 200 can move to the outside of the distal end of the puncture needle 20 during the flipping process.

The puncture needle 20 is withdrawn so that the puncture needle 20 is withdrawn into the instrument channel of the endoscope, and the driving tube 300 is withdrawn into the puncture needle 20; then the puncture needle 20 is withdrawn together with the driving tube 300, leaving the traction line 100 in the instrument channel.

The traction line 100 is pulled, for example, the traction line 100 is pulled from the proximal end of the traction line 100, so that the anchoring body 200 is first attached to the inner wall of the gallbladder, and then the gallbladder is pulled to the outer wall of the intestine to achieve the anchoring of the gallbladder.

Referring to Figures 4a to 4d, after the operation is completed, the grasping forceps can be extended into the first tissue 30 through the instrument channel of the endoscope, and the proximal end of the recovery line 400 can be grasped under the scope and withdrawn. The anchoring body 200 is flipped under the pull of the recovery line 400, so that one end of the anchoring body 200 enters the puncture channel, and enters the first tissue 30 along the puncture channel, and then moves out of the body along the instrument channel of the endoscope.

In the embodiment of the present invention, since the traction line 100 is a flexible member, there is no resilience forth between the anchoring body 200 and the traction line 100, that is, the anchoring body 200 does not have a resilience forth to restore to the anchor position. Compared with the above-mentioned example where the traction line 100 is of a hard material, one end of the anchoring body 200 can be easily flipped to the opening at one end of the puncture channel under the pulling of the recovery line 400, and continue to enter the puncture channel, reducing or avoiding the collision of one end of the anchoring body 200 with the inner wall of the target tissue 40 when reaching the opening at one end of the puncture channel, thereby damaging the target tissue 40.

In addition, when the anchoring body 200 moves along the puncture channel, since the anchoring body 200 does not have a resilience forth to restore to the anchor position, the friction between the anchoring body 200 and the inner wall of the puncture channel is reduced, thereby reducing the damage to the inner wall of the puncture channel, i.e., the target tissue 40 and the first tissue 30.

It should be noted that during the withdrawal of the anchoring body 200 along the puncture channel, the length direction of the anchoring body 200 and the extension direction of the puncture channel may be parallel or may have an angle. For example, the angle may be less than or equal to 30°, for example, the angle may be equal to a suitable angle value such as 10°, 15°, 20° or 30°.

The extension direction of the puncture channel can be shown as shown in the direction a in Figures 4a to 4d, and the length direction of the anchoring body 200 is the extension direction of the line between the proximal end of the anchoring body 200 and the distal end of the anchoring body 200.

In some examples, the driving tube 300 may be sleeved on the traction line 100, and the anchoring body 200 is located at the distal end of the driving tube 300, that is, the driving tube 300 is only sleeved on the traction line 100, and the anchoring body 200 is exposed outside the driving tube 300 and is located at the distal end of the driving tube 300.

When the anchoring body 200 is located in the puncture needle 20, the proximal end of the anchoring body 200 (see 200a in Figure 2) abuts against the distal end (300b) of the driving tube 300, so that when the anchoring body 200 extends out of the puncture needle 20, for example, when the proximal end of the anchoring body 200 is pushed out of the distal end of the puncture needle 20, the anchoring body 200 can be flipped under the pushing of the driving tube 300 and the pulling of the traction line 100.

For example, during the implantation of the anchor 10, the driving tube 300 may first push the distal end of the anchoring body 200 (see 200b in Figure 2) from the proximal end of the puncture needle 20 to gradually enter the needle channel of the puncture needle 20, and then the distal end of the driving tube 300 abuts the proximal end of the anchoring body 200, so that the anchoring body 200 is pushed toward the distal end of the puncture needle 20. When the anchoring body 200 extends out of the distal end of the puncture needle 20, for example, when the proximal end of the anchoring body 200 just reaches the distal end of the puncture needle 20, the driving tube 300 may continue to abut the anchoring body 200, and simultaneously the traction line 100 is pulled, so that the anchoring body 200 can achieve stable flipping under the assistance of the pushing of the driving tube 300 and the pulling of the traction line 100.

Of course, in some examples, when the anchoring body 200 extends out of the distal end of the puncture needle 20, the driving tube 300 may not be needed to abut the anchoring body 200, and the anchoring body 200 can be flipped under the pulling of the traction line 100.

When the anchoring body 200 is located in the puncture needle 20, the length direction of the anchoring body 200 can be parallel to the extension direction of the puncture needle 20, or it can have an angle with the extension direction of the puncture needle 20. However, because the traction line 100 is arranged as a flexible member, there is no resilience forth between the traction line 100 and the anchoring body 200, so that the anchoring body 200 will not excessively resist the side wall of the puncture needle 20 when moving in the puncture needle 20, and only move along the side wall of the puncture needle 20, thereby reducing the friction between the anchoring body 200 and the puncture needle 20, ensuring that the anchoring body 200 moves stably along the puncture needle 20 under the drive of the driving tube 300, improving the implantation stability and implantation efficiency of the anchoring body 200.

In some other examples, the driving tube 300 can be sleeved on the outer periphery of the traction line 100 and the anchoring body 200 (not shown in the figure), that is, the anchoring body 200 and the traction line 100 are both located in the driving tube 300. During the implantation process of the anchor 10, the distal end of the driving tube 300 can gradually enter the needle channel of the puncture needle 20 from the proximal end of the puncture needle 20, so that the anchoring body 200 and a part of the traction line 100 enter the needle channel of the puncture needle 20, and then the driving tube 300 is pushed, so that the driving tube 300 is advanced toward the distal end of the puncture needle 20. When the anchoring body 200 reaches the distal end of the puncture needle 20 under the driven by the driving tube 300, for example, when the distal end of the driving tube 300 is flush with the distal end of the anchoring body 200, and the distal end of the driving tube 300 just reaches the distal end of the puncture needle 20, it indicates that the distal end of the anchoring body 200 reaches the distal end of the puncture needle 20, and then the driving tube 300 can be withdrawn, so that the anchoring body 200 is exposed from the driving tube 300, and the proximal end of the anchoring body 200 can be abutted against the distal end of the driving tube 300. Then the driving tube 300 is continuously pushed toward the distal end of the puncture needle 20, so that the anchoring body 200 is exposed from the puncture needle 20 under the push of the driving tube 300. When the proximal end of the anchoring body 200 just reaches the distal end of the puncture needle 20, the driving tube 300 can continue to abut against the anchoring body 200, and while the traction line 100 is pulled, so that the anchoring body 200 can achieve stable flipping under the assistance of the pushing of the driving tube 300 and the pulling of the traction line 100.

Of course, in some examples, a part of the anchoring body 200 can be arranged in the driving tube 300, and the other part can be exposed outside the driving tube 300. When the distal end of the anchoring body 200 reaches the distal end of the puncture needle 20, the driving tube 300 can be withdrawn so that the proximal end of the anchoring body 200 abuts against the distal end of the driving tube 300, and the driving tube 300 is continuously pushed toward the distal end of the puncture needle 20, so that the anchoring body 200 is exposed from the puncture needle 20 under the push of the driving tube 300. Until the proximal end of the anchoring body 200 just reaches the distal end of the puncture needle 20, the driving tube 300 can continue to abut against the anchoring body 200, so that the anchoring body 200 can achieve stable flipping under the assistance of the pushing of the driving tube 300 and the pulling of the traction line 100.

It can be understood that in the above example, since the traction line 100 is a flexible member, there is no resilience forth between the traction line 100 and the anchoring body 200, so that the anchoring body 200 will not tightly contact the side wall of the driving tube 300, and the contact degree between the anchoring body 200 and the inner wall of the driving tube 300 can allow the anchoring body 200 and the driving tube 300 to move relative to each other when the driving tube 300 is withdrawn. It is only necessary to ensure that the anchoring body 200 and the traction line 100 can be pushed to the distal end of the puncture needle 20 together during the advancement of the driving tube 300.

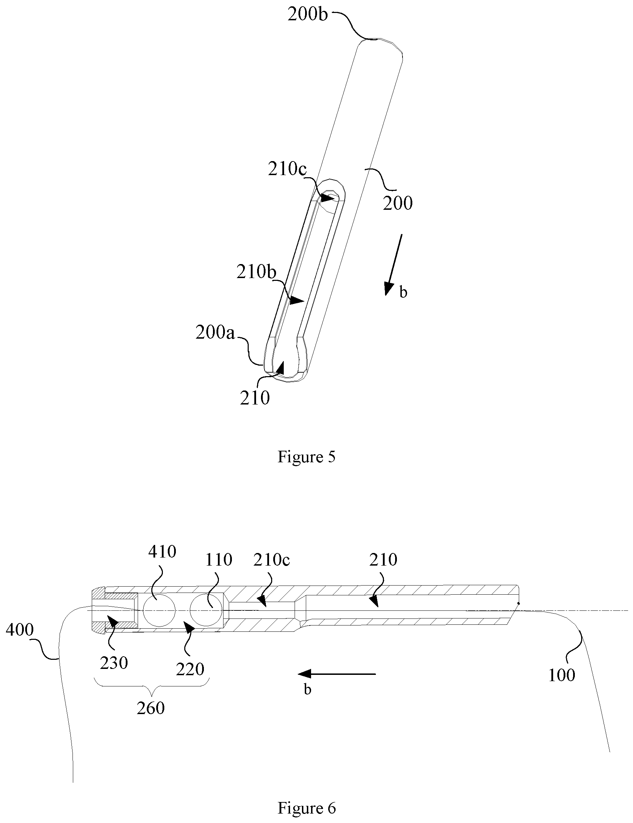

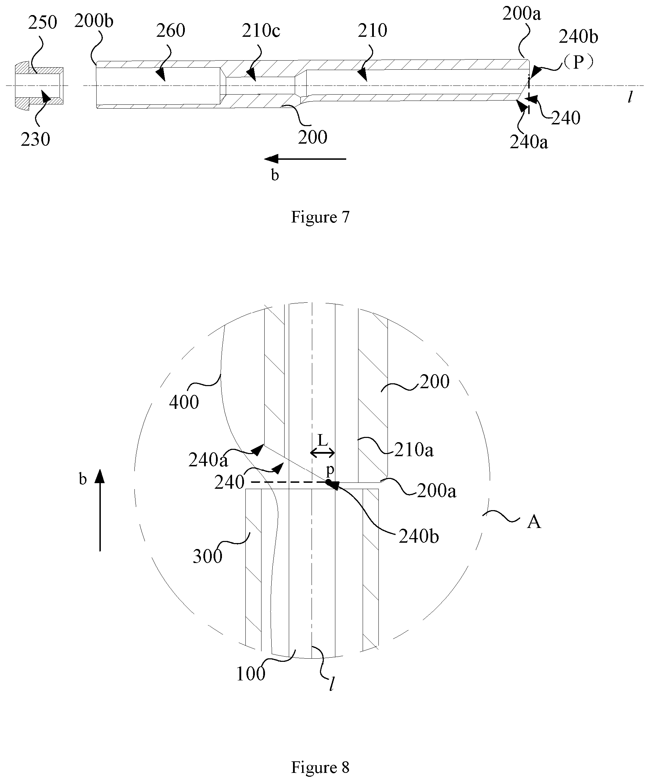

Figure 5 is a structural schematic diagram of one of the anchors provided in an embodiment of the present invention, Figure 6 is an assembly sectional view of the anchor, the traction line and the recovery line in Figure 5, Figure 7 is a sectional view of the anchor in Figure 6, and Figure 8 is a partial enlarged view of part A in Figure 2. Referring to Figures 5 to 8, in some examples, a limiting groove 210 and a side notch 210b connected to the limiting groove 210 can be formed on the anchoring body 200. The limiting groove 210 and the side notch 210b both extend along the length direction of the anchoring body 200, and one end of the limiting groove 210 and the side notch 210b both run through the proximal end of the anchoring body 200. The length direction of the anchoring body 200 can be shown as direction b in Figures 5 to 8.

It can be understood that the side notch 210b can be the opening of the limiting groove 210 on the side wall of the anchoring body 200. For the convenience of description, the two opposite ends of the limiting groove 210 along the length direction can be respectively referred to as the first end and the second end, wherein the end that runs through the proximal end of the anchoring body 200 is referred to as the first end, and the end away from the proximal end of the anchoring body 200 is referred to as the second end.

The first end of the limiting groove 210 runs to the proximal end of the anchoring body 200, which indicates that the first end of the limiting groove 210 is provided with an end notch, the end notch communicates with the side notch 210b, which face in different orientations.

When the proximal end of the anchoring body 200 is located at the puncture needle 20, at least part of the traction line 100 is located in the limiting groove 210, and extends out of the anchoring body 200 from the end notch of the limiting groove 210, and extends into the driving tube 300. During the process of the traction line 100 pulling the anchoring body 200 to flip, a part of the traction line 100 is detached from the anchoring body 200 from the side notch 210b.

For example, when the traction line 100 is fixed, it can be fixed at any position in the limiting groove 210, or it can be fixed at a position in the anchoring body 200 other than the limiting groove 210, as long as part of the traction line 100 is received in the limiting groove 210.

In the embodiment of the present invention, a limiting groove 210 is provided on the anchoring body 200 to limit the traction line 100 in the radial direction of the anchoring body 200, so that when the anchoring body 200 is in the puncture needle 20, the traction line 100 is located in the anchoring body 200, which plays a good receiving role for the traction line 100 and avoids the traction line 100 from rubbing against the puncture needle 20 and accumulating.

In some other examples, a mounting hole perpendicular to the length direction can be formed in the anchoring body 200, so that the distal end of the traction line 100 is fixed in the anchoring body 200 and extends to the outside of the anchoring body 200 through the mounting hole. When the proximal end of the anchoring body 200 is located in the puncture needle 20, the traction line 100 extends along the outer wall of the anchoring body 200 into the driving tube 300.

As shown in Figures 6 to 8, in order to achieve better flipping of the anchoring body 200, in some examples, the end (i.e., the first end) of the limiting groove 210 located at the proximal end of the anchoring body 200 is provided with an avoidance opening 240, one end of the avoidance opening 240 extends to the side notch 210b, and the other end of the avoidance opening 240 extends to the side of the axis *l* of the limiting groove 210 that is away from the side notch 210b. It should be noted that the axis of the limiting groove 210 is consistent with the axis of the anchoring body 200, that is, the limiting groove 210 is coaxially arranged with the anchoring body 200.

For the convenience of description, the portion of the limiting groove 210 having the avoidance opening 240 in the length direction can be referred to as the first portion, and the other portions of the limiting groove 210 in the length direction can be referred to as the second portion. The angle at which the groove wall of the second portion of the limiting groove 210 extends circumferentially around the axis *l* is the second angle, and the angle at which the groove wall of the first portion of the limiting groove 210 extends circumferentially around the axis *l* is the first angle.

The first angle and the second angle are both less than 360°, so that a side notch 210b is formed on the side wall of the anchoring body 200, that is, the limiting groove 210 is not closed in the circumferential direction. The first angle is smaller than the second angle, so that the first end of the limiting groove 210 forms an avoidance opening 240.

In some examples, the first angle can be reduced in the direction from the second portion of the limiting groove 210 to the proximal end of the anchoring body 200, and the slope of the reduction is equal, so that the end surface of the avoidance opening 240 communicating with the side notch is formed as an inclined plane. Of course, in some other examples, when the slopes of the reduction of the first angle in the direction from the second portion of the limiting groove 210 to the proximal end of the anchoring body 200 are different, for example, the slopes of the reduction gradually increases, so that the end surface of the avoidance opening 240 communicating with the side notch is formed as an arc surface.

Of course, in some examples, the first angle remains unchanged in the direction from the second portion of the limiting groove 210 to the proximal end of the anchoring body 200, then the end surface of the avoidance opening 240 communicating with the side notch is a vertical surface perpendicular to the side notch, and the avoidance opening 240 has a horizontal surface perpendicular to the vertical surface, that is, the entire end surface of the avoidance opening 240 is a right angle surface. The embodiment of the present invention does not limit the shape of the end surface of the avoidance opening 240.

In addition, the other end of the avoidance opening 240 extends to the side of the axis *l* of the limiting groove 210 that is away from the side notch 210b, that is, the first angle of the end of the first portion of the limiting groove 210 located at the proximal end of the anchoring body 200 is less than 180°, that is, the end groove wall of the first portion of the limiting groove 210 located at the proximal end of the anchoring body 200 extends circumferentially around the axis *l* to a plane below that where the axis *l* is located, that is, the other end of the avoidance opening 240 has a first distance from the axis *l* of the limiting groove 210 in the radial direction of the anchoring body 200 (refer to L in Figure 8). It can be understood that the plane where the axis *l* is located is arranged opposite to the groove bottom 210a.

By forming an avoidance opening 240 at the first end of the limiting groove 210, and one end of the avoidance opening 240 extends to the side notch 210b, and the other end extends to the side of the axis *l* of the limiting groove 210 away from the side notch 210b, so that the end surface of the proximal end of the anchoring body 200, except for the avoidance opening 240, abuts the distal end of the driving tube 300, and the end of the avoidance opening 240 away from the side notch 210b serves as the flip fulcrum P of the anchoring body 200.

When the proximal end of the anchoring body 200 reaches or is about to reach the distal end of the puncture needle 20, the traction line 100 is pulled, so that the flip fulcrum P is subjected to the pushing force of the distal end of the driving tube 300, and because the flip fulcrum P has a first distance from the axis *l* of the limiting groove 210, when the anchoring body 200 is pulled by the traction line 100, the flip fulcrum P is pushed by the distal end of the driving tube 300, thereby forming a flipping torque, so that the anchoring body 200 can achieve better flipping.

In some examples, there is a gap between the other end of the avoidance opening 240 (i.e., the flip fulcrum P) and the groove bottom 210a of the limiting groove 210. In other words, the other end of the avoidance opening 240 does not extend to the groove bottom 210a of the limiting groove 210, so that the proximal end of the anchoring body 200 reserves a portion of the end surface perpendicular to the axis *l*, so that the distal end of the driving tube 300 can stably abut against the end notch of the limiting groove 210 without entering the limiting groove 210, or shaking on the end notch of the limiting groove 210, so that the driving tube 300 can push the anchoring body 200 to move stably along the puncture needle 20, the driving tube 300 can stably abut against the flip fulcrum P, and the anchoring body 200 can be pulled to flip stably under the pulling of the traction line 100.

The groove bottom 210a of the limiting groove 210 is arranged opposite to the side notch 210b.

Exemplarily, the distance between the other end of the avoidance opening 240 and the groove bottom 210a of the limiting groove 210 may be less than or equal to 5 mm. For example, the distance may be a suitable value such as 1 mm, 3 mm or 5 mm. The embodiment of the present invention does not limit the distance.

In some examples, when the proximal end of the anchoring body 200 is located at the puncture needle 20, the traction line 100 may extend along the axis *l* of the limiting groove 210, so that the traction line 100 pulls the anchoring body 200 along the axis *l* of the limiting groove 210, so that the anchoring body 200 is flipped.

In some other examples, when the proximal end of the anchoring body 200 is located at the puncture needle 20, the traction line 100 may be located between the axis *l* of the limiting groove 210 and the side notch 210b. For the convenience of description, the side of the limiting groove 210 located on the axis *l* toward the side notch 210b can be referred to as the first side, and the side of the limiting groove 210 located on the axis *l* toward the flip fulcrum P (or the groove bottom 210a) can be referred to as the second side. The traction line 100 is arranged on the first side of the limiting groove 210 to deviate from the second side, that is, the flip fulcrum P. In this way, the traction line 100 pulls the anchoring body 200 along the side away from the flip fulcrum P, which can increase the flipping torque of the anchoring body 200, so that the anchoring body 200 can achieve better flipping under the pulling of the traction line 100 and the pushing of the driving tube 300.

In some examples, the distal end of the traction line 100 can be fixed at any position of the limiting groove 210, for example, it can be fixed inside the second end of a single limiting groove 210.

As shown in Figure 6, in some other examples, a line placement cavity 220 may be formed on the anchoring body 200.

The line placement cavity 220 communicates with the groove cavity of the limiting groove 210 through a penetration hole 210c. The distal end of the traction line 100 is limited in the line placement cavity 220, and the traction line 100 extends from the penetration hole 210c into the limiting groove 210.

For example, the line placement cavity 220 and the limiting groove 210 can be arranged at intervals along the length direction of the anchoring body 200, so that the part of the traction line 100 located in the anchoring body 200 extends along the length direction of the anchoring body 200.

The penetration hole 210c is located in the anchoring body 200 and extends along the length direction of the anchoring body 200. The arrangement of the penetration hole 210c can better limit the specific position of the traction line 100 on the anchoring body 200 in the radial direction, and simplify the matching structure of the anchoring body 200 and the traction line 100. For example, the penetration hole 210c can be arranged on the axis *l* of the limiting groove 210, that is, the axis *l* of the penetration hole 210c coincides with the axis *l* of the limiting groove 210. In this way, when the distal end of the traction line 100 is fixed in the line placement cavity 220 and the traction line 100 enters the limiting groove 210 along the penetration hole 210c, the traction line 100 can extend along the axis *l* of the limiting groove 210. Similarly, when the penetration hole 210c is arranged on the extension area of the first side of the limiting groove 210, the traction line 100 is limited to the extension area of the first side of the limiting groove 210.

In some examples, the distal end of the traction line 100 can be fixed in the line placement cavity 220 by bonding or welding, and so on.

In some other examples, the distal end of the traction line 100 is provided with a first limiting portion 110, and the first limiting portion 110 is clamped in the line placement cavity 220, so that the distal end of the traction line 100 is limited in the line placement cavity 220.

It can be understood that the width dimension of the first limiting portion 110 is greater than the opening width dimension of the line placement cavity 220, so that the first limiting portion 110 is clamped in the line placement cavity 220, and the distal end of the traction line 100 is limited in the line placement cavity 220 and will not detach from the notch of the line placement cavity 220. In addition, the outer contour dimension of the first limiting portion 110 is greater than the first orifice of the penetration hole 210c, so that the first limiting portion 110 will not enter the limiting groove 210 from the penetration hole 210c, ensuring that the first limiting portion 110, i.e., the distal end of the traction line 100, is limited in the line placement cavity 220.

In some examples, the first limiting portion 110 can be movable in the line placement cavity 220 or fixed in the line placement cavity 220, and the embodiments of the present invention are not limited to this.

For example, the first limiting portion 110 can be an integral part with the distal end of the traction line 100, for example, the first limiting portion 110 can be a knot at the distal end of the traction line 100. In some examples, the first limiting portion 110 can also be a block or spherical limiting structure fixed at the distal end of the traction line 100.

In some examples, when the anchoring body 200 flips to the anchoring position, the position where the traction line 100 is detached from the anchoring body 200 is located at the center position of the anchoring body 200 along the length direction.

It can be understood that when the anchoring body 200 flips to the anchoring position, a part of the traction line 100 is located inside the anchoring body 200, and the other part is detached from the anchoring body 200, that is, the other part is located outside the anchoring body 200, then the position where the traction line 100 is detached from the anchoring body 200 (as shown in M in Figure 3) refers to the position on the anchoring body 200 where the turning point between the part of the traction line 100 located inside the anchoring body 200 and the part of the traction line 100 located outside the anchoring body 200 corresponds to.

By arranging the position where the traction line 100 is detached from the anchoring body 200 at the center position of the anchoring body 200 along the length direction, the anchoring body 200 will not deviate from the anchoring position during the pulling process of the traction line 100, that is, the angle between the anchoring body 200 and the traction line 100 will not deviate from the anchoring angle, ensuring that the anchoring body 200 can stably fit with the inner wall of the target tissue 40 during the pulling process of the traction line 100 without causing damage to the target tissue 40.

It is understandable that when the anchoring body 200 is flipped to the anchoring position, the position where the traction line 100 is detached from the anchoring body 200 is the second end of the limiting groove 210. Therefore, the second end of the limiting groove 210 can be extended to the center position of the anchoring body 200, so that the position where the traction line 100 is detached from the anchoring body 200 is the center position of the anchoring body 200.

In some examples, the distal end of the traction line 100 can be directly fixed to the center position of the anchoring body 200, for example, it can be fixed to the second end of the limiting groove 210.

In some other examples, the distal end of the traction line 100 can be located between the center position of the anchoring body 200 and the distal end of the anchoring body 200. For example, the distal end of the traction line 100 is confined in the above-mentioned line placement cavity 220. Since the above-mentioned line placement cavity 220 is located on the side of the limiting groove 210 toward the distal end of the anchoring body 200, for example, the line placement cavity 220 is located between the center position of the anchoring body 200 and the distal end of the anchoring body 200, the distal end of the traction line 100 deviates from the center position of the anchoring body 200, for example, it can be located between the center position of the anchoring body 200 and the distal end of the anchoring body 200, and the traction line 100 extends toward the proximal end of the anchoring body 200.

In some examples, the fixed end of the recovery line 400 is fixed to the center position of the anchoring body 200 along the length direction, or the fixed end of the recovery line 400 is located between the center position of the anchoring body 200 and the distal end of the anchoring body 200 (as shown in Figure 6).

Referring to Figure 4a to Figure 4d, the recovery line 400 extends toward the distal end of the anchoring body 200 and extends out of the anchoring body 200 from the distal end of the anchoring body 200, and the recovery line 400 is configured to drive the distal end of the anchoring body 200 to flip toward the puncture channel.

In the above example, the traction line 100 extends toward the proximal end of the anchoring body 200, and when the traction line 100 and the anchoring body 200 are located at the puncture needle 20, the traction line 100 extends out of the anchoring body 200 from the proximal end of the anchoring body 200, and when the anchoring body 200 extends out of the puncture needle 20 and flips to the anchoring position, the traction line 100 detaches from the anchoring body 200 from the center position of the anchoring body 200.

Therefore, by extending the recovery line 400 toward the distal end of the anchoring body 200 and extending out the anchoring body 200 from the distal end of the anchoring body 200, the recovery line 400 can drive the distal end of the anchoring body 200 to flip toward the puncture channel without interfering with the traction line 100 located on the other side of the anchoring body 200, thereby avoiding the accumulation of the traction line 100 and affecting the distal end of the anchoring body 200 from entering the puncture channel, thereby improving the recovery efficiency of the anchoring body 200.

In some examples, the fixed end of the recovery line 400 can be directly fixed to the distal end of the anchoring body 200 or the side wall near the distal end.

As shown in Figure 6, in some other examples, a limiting channel 230 may be formed on the anchoring body 200, the limiting channel 230 communicates with the line placement cavity 220, and one end of the limiting channel 230 passes through the distal end of the anchoring body 200. It can be understood that the limiting channel 230 is located on the side of the line placement cavity 220 away from the limiting groove 210.

The fixed end of the recovery line 400 is fixed in the line placement cavity 220, and the recovery line 400 extends out of the distal end of the anchoring body 200 through the limiting channel 230.

The limiting channel 230 is arranged along the length direction of the anchoring body 200. The arrangement of the limiting channel 230 can better limit the specific position of the recovery line 400 on the anchoring body 200 in the radial direction, and simplify the matching structure of the anchoring body 200 and the recovery line 400. For example, the limiting channel 230 can be arranged on the axis *l* of the anchoring body 200. In this way, when the fixed end of the recovery line 400 is fixed in the line placement cavity 220 and the recovery line 400 extends from the limiting channel 230 out the distal end of the anchoring body 200, the recovery line 400 can extend along the axis *l* of the anchoring body 200, so that the anchoring body 200 can be easily flipped.

In some examples, the fixed end of the recovery line 400 can be fixed in the line placement cavity 220 by bonding or welding.

In other examples, the fixed end of the recovery line 400 is provided with a second limiting portion 410, and the second limiting portion 410 is clamped in the line placement cavity 220 to limit the fixed end of the recovery line 400 in the line placement cavity 220.

It can be understood that the width dimension of the second limiting portion 410 is greater than the opening width dimension of the line placement cavity 220, so that the second limiting portion 410 is clamped in the line placement cavity 220, and the fixed end of the recovery line 400 is limited in the line placement cavity 220, and will not detach from the notch of the line placement cavity 220.

In addition, the outer contour dimension of the second limiting portion 410 is greater than the dimension of the first through hole 250a, so that the second limiting portion 410 will not enter the limiting channel 230 from the first through hole 250a, ensuring that the second limiting portion 410 is limited in the line placement cavity 220, that is, the fixed end of the recovery line 400 is limited in the line placement cavity 220.

In some examples, the second limiting portion 410 can be movable in the line placement cavity 220, or can be fixed in the line placement cavity 220, and the embodiment of the present invention does not limit this.

Exemplarily, the second limiting portion 410 can be an integral part with the fixed end of the recovery line 400, for example, the second limiting portion 410 can be a knot at the fixed end of the recovery line 400. In some examples, the second limiting portion 410 can also be a block or spherical limiting structure fixed to the fixed end of the recovery line 400.

In some examples, the fixed end of the recovery line 400 is connected to the second limiting portion 410 at the distal end of the traction line 100, and the second limiting portion 410 is clamped in the line placement cavity 220 to limit the fixed end of the recovery line 400 in the line placement cavity 220.

It can be understood that in this example, the recovery line 400 and the traction line 100 share a first limiting portion 110 to simplify the structure of the anchor 10. In this example, the outer contour size of the first limiting portion 110 needs to be larger than the size of the first through hole 250a, so that the first limiting portion 110 will not enter the limiting channel 230 from the first through hole 250a, so that the fixed ends of the traction line 100 and the recovery line 400 are both limited in the line placement cavity 220.

Exemplarily, the first limiting portion 110, the traction line 100 and the recovery line 400 can be an integrally formed one-piece to simplify the structure of the entire anchor 10 and improve the assembly efficiency of the anchor 10.

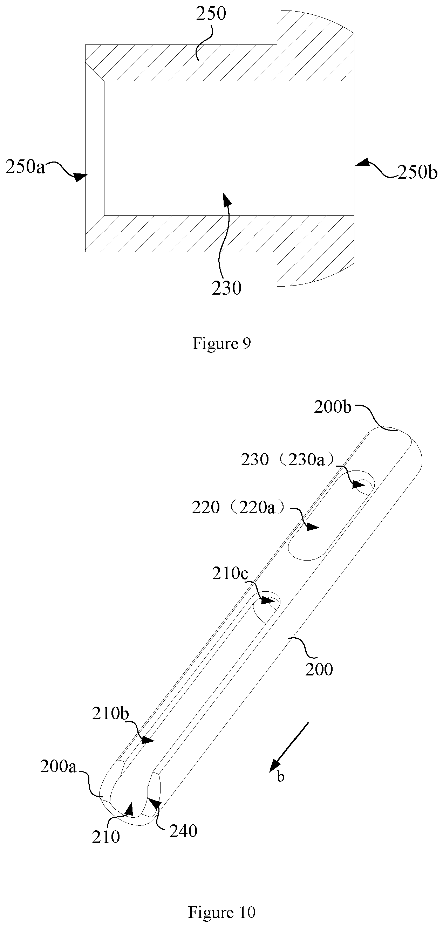

Figure 9 is a structure schematic diagram of the end cap in Figure 8. Referring to Figures 5 to 9, in some examples, a mounting channel 260 is formed on the anchoring body 200, one end of the mounting channel 260 communicates with the penetration hole 210c, and the other end of the mounting channel 260 runs through to the distal end of the anchoring body 200. For example, the mounting channel 260 is located on the side of the limiting groove 210 facing the distal end of the anchoring body 200, and the penetration hole 210c is located between the mounting channel 260 and the limiting groove 210, and communicates the mounting channel 260 with the limiting groove 210.

In some examples, the distal end of the anchoring body 200 has an end cap 250, and the end cap 250 is embedded in the mounting channel 260 from the distal end of the anchoring body 200. One end of the end cap 250 is spaced from the penetration hole 210c, and a line placement cavity 220 is formed between one end of the end cap 250 and the penetration hole 210c. In addition, the inner cavity of the end cap 250 forms the above-mentioned limiting channel 230.

For example, the penetration hole 210c has a first orifice and a second orifice that are arranged oppositely along the length direction of the anchoring body 200, wherein the first orifice faces the limiting groove 210 and communicates with the limiting groove 210, and the end surface where the second orifice is located is spaced apart from one end of the end cap 250, and the end surface where the second orifice is located and one end of the end cap 250 form a line placement cavity 220.

For example, the end cap 250 has a hollow structure inside, and both ends of the end cap 250 have through holes. For the convenience of description, the through hole at one end of the end cap 250 facing the line placement cavity 220 is called the first through hole 250a, and the through hole at one end of the end cap 250 away from the line placement cavity 220 is called the second through hole 250b. The first through hole 250a is used to communicate the inner cavity of the end cap 250 (i.e., the limiting channel 230) with the line placement cavity 220, and the second through hole 250b is used to communicate the inner cavity of the end cap 250 (i.e., the limiting channel 230) with the outside of the anchoring body 200, so that the recovery line 400 extends out of the anchoring body 200 through the first through hole 250a, the inner cavity and the second through hole 250b successively.

It can be understood that the inner cavity can be used as the limiting channel 230, the first through hole 250a is an opening at one end of the limiting channel 230, and the second through hole 250b is an opening at the other end of the limiting channel 230.

By arranging an mounting channel 260 on the anchoring body 200, in which the mounting channel 260 extends to the distal end of the anchoring body 200, and arranging an end cap 250 at the distal end of the anchoring body 200 to form a line placement cavity 220 and a limiting channel 230, the structural arrangement of the anchoring body 200 is simplified, and the distal end of the traction line 100 and the fixed end of the recovery line 400 are conveniently assembled in the line placement cavity 220.

Figure 10 is a structural schematic diagram of another anchor provided in an embodiment of the present invention. As shown in Figure 10, in some other examples, the side wall of the anchoring body 200 is recessed inwardly to form a line placement groove 220a, and the groove cavity of the line placement groove 220a is configured as a line placement cavity 220.

In this example, the limiting channel 230 is a limiting hole 230a formed on the anchoring body 200, and the two ends of the limiting hole 230a respectively run to the line placement groove 220a and the distal end of the anchoring body 200, so that the free end of the recovery line 400 extends from the line placement groove 220a through the limiting hole 230a to the outside of the anchoring body 200.

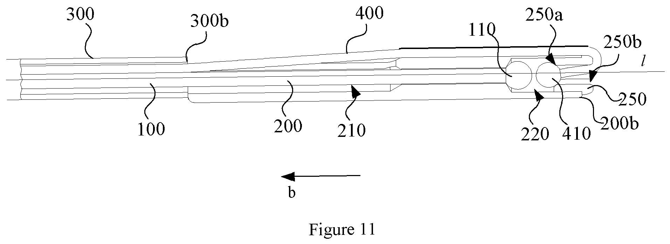

Figure 11 is a partial structural schematic diagram of the anchor provided by an embodiment of the present invention. In some examples, at least part of the recovery line 400 is located in the driving tube 300, so as to enter the target tissue 40 under the drive of the driving tube 300.

For example, the fixed end of the recovery line 400 is located in the line placement cavity 220 of the anchoring body 200, and the recovery line 400 extends out of the distal end of the anchoring body 200 along the limiting channel 230, and folds back to enter the driving tube 300. It is understandable that by embedding at least part of the recovery line 400 in the driving tube 300, the recovery line 400 can be tightened in the anchor 10, so that when the driving tube 300 drives the anchoring body 200 to move along the puncture needle 20, the recovery line 400 can move forward with the driving tube 300, thereby avoiding the situation that the recovery line 400 is accumulated at the distal end of the driving tube 300 when the driving tube 300 is inadvertently withdrawn, which affecting the advancement and flipping of the anchoring body 200.

In addition, by providing the free end of the recovery line 400 in the driving tube 300, it is avoided that when the driving tube 300 is inadvertently withdrawn, the recovery line 400 rubs against the inner wall of the needle channel of the puncture needle 20, causing the recovery line 400 to accumulate at the distal end of the driving tube 300, thereby affecting the advancement and flipping of the anchoring body 200.

The above specific embodiments further explain in detail the purpose, technical solution and beneficial effects of the embodiments of the present invention. It should be understood that the above embodiment is only a specific embodiment of the embodiments of the present invention, and is not used to limit the protection scope of the embodiments of the present invention. Any modifications, equivalent replacements, improvements, etc. made on the basis of the technical solution of the embodiments of the present invention should be included in the protection scope of the embodiments of the present invention.

## Claims

1. An anchor (10), **characterized by** comprising:
a traction line (100), wherein the traction line (100) is a flexible member;
an anchoring body (200), connected to a distal end of the traction line (100) and capable of being pulled by the traction line (100);
a driving tube (300), at least sleeved on at least a portion of the traction line (100) and configured to drive the anchoring body (200) to move along a puncture needle (20) so that the anchoring body (200) enters a target tissue (40); and
a recovery line (400), wherein the recovery line (400) is connected to the anchoring body (200), and the recovery line (400) is configured to pull the anchoring body (200) so that the anchoring body (200) is separated from the target tissue (40) along a puncture channel of the target tissue (40), wherein the puncture channel is a channel formed by puncturing the target tissue (40) by the puncture needle (20).

2. The anchor (10) according to claim 1, wherein the driving tube (300) is sleeved on the traction line (100), and the anchoring body (200) is located outside a distal end of the driving tube (300); and
when the anchoring body (200) is located in the puncture needle (20), a proximal end of the anchoring body (200) abuts the distal end of the driving tube (300), so that when the anchoring body (200) extends out of the puncture needle (20), the anchoring body (200) can be flipped under pushing of the driving tube (300) and pulling of the traction line (100).

3. The anchor (10) according to claim 2, wherein a limiting groove (210) and a side notch (210b) communicated with the limiting groove (210) are formed on the anchoring body (200), the limiting groove (210) and the side notch (210b) both extend along a length direction of the anchoring body (200), and one ends of the limiting groove (210) and the side notch (210b) both run through a proximal end of the anchoring body (200),
wherein when the proximal end of the anchoring body (200) is located at the puncture needle (20), at least part of the traction line (100) is located in the limiting groove (210), and during a process of the traction line (100) pulling the anchoring body (200) to flip, a part of the traction line (100) detaches from the anchoring body (200) through the side notch (210b).

4. The anchor (10) according to claim 3, wherein an avoidance opening (240) is formed at one end of the limiting groove (210) located at the proximal end of the anchoring body (200), one end of the avoidance opening (240) extends to the side notch (210b), and the other end of the avoidance opening (240) extends to a side of an axis of the limiting groove (210) away from the side notch (210b).

5. The anchor (10) according to claim 4, wherein a gap is provided between the other end of the avoidance opening (240) and a groove bottom (210a) of the limiting groove (210); and
the groove bottom (210a) of the limiting groove (210) is arranged opposite to the side notch (210b).

6. The anchor (10) according to claim 4, wherein when the proximal end of the anchoring body (200) is located at the puncture needle (20), the traction line (100) extends along the axis of the limiting groove (210); or
when the proximal end of the anchoring body (200) is located at the puncture needle (20), the traction line (100) is located between the axis of the limiting groove (210) and the side notch (210b).

7. The anchor (10) according to claim 1, wherein when the anchoring body (200) is flipped to an anchoring position, a position where the traction line (100) is detached from the anchoring body (200) is located at a center position of the anchoring body (200) along a length direction.

8. The anchor (10) according to claim 7, wherein one end of the recovery line (400) is located at the center position of the anchoring body (200) along the length direction; or
one end of the recovery line (400) is located between the center position of the anchoring body (200) along the length direction and a distal end of the anchoring body (200),
wherein the distal end of the traction line (100) is located between the center position and the distal end of the anchoring body (200), and the traction line (100) extends toward the proximal end of the anchoring body (200); the recovery line (400) extends toward the distal end of the anchoring body (200), and extends out of the anchoring body (200) from the distal end of the anchoring body (200); and the recovery line (400) is configured to drive the distal end of the anchoring body (200) to flip toward the puncture channel.

9. The anchor (10) according to any one of claims 3 to 6, wherein a line placement cavity (220) is formed on the anchoring body (200),
wherein the line placement cavity (220) communicates with a groove cavity of the limiting groove (210) through a penetration hole (210c), the distal end of the traction line (100) is limited in the line placement cavity (220), and the traction line (100) extends from the penetration hole (210c) into the limiting groove (210),
preferably, a first limiting portion (110) is formed at the distal end of the traction line (100), and the first limiting portion (110) is embedded in the line placement cavity (220) so that the distal end of the traction line (100) is limited in the line placement cavity (220),
preferably, the anchoring body (200) also forms a limiting channel (230), the limiting channel (230) communicates with the line placement cavity (220) of the anchoring body (200), and one end of the limiting channel (230) runs through a distal end of the anchoring body (200); and
one end of the recovery line (400) is fixed in the line placement cavity (220), and the recovery line (400) extends out of the distal end of the anchoring body (200) through the limiting channel (230).

10. The anchor (10) according to claim 9, wherein a second limiting portion (410) is formed at one end of the recovery line (400), and the second limiting portion (410) is clamped in the line placement cavity (220) so that one end of the recovery line (400) is limited in the line placement cavity (220).

11. The anchor (10) according to claim 9, wherein one end of the recovery line (400) is connected to the first limiting portion (110) at the distal end of the traction line (100), and the first limiting portion (110) is embedded in the line placement cavity (220) so that one end of the recovery line (400) is limited in the line placement cavity (220).

12. The anchor (10) according to claim 9, wherein a side wall of the anchoring body (200) is recessed inwardly to form a line placement groove (220a), and a groove cavity of the line placement groove (220a) is configured as the line placement cavity (220); and
the limiting channel (230) is a limiting hole (230a) formed on the anchoring body (200), and two ends of the limiting hole (230a) respectively run through the line placement groove (220a) and the distal end of the anchoring body (200).

13. The anchor (10) according to claim 9, wherein a mounting channel (260) is formed in the anchoring body (200), one end of the mounting channel (260) communicates with the penetration hole (210c), and the other end of the mounting channel (260) runs to the distal end of the anchoring body (200),
wherein the distal end of the anchoring body (200) has an end cap (250), the end cap (250) is embedded in the mounting channel (260) from the distal end of the anchoring body (200), and one end of the end cap (250) is spaced from the penetration hole (210c); and
the line placement cavity (220) is formed between one end of the end cap (250) and the penetration hole (210c), an inner cavity of the end cap (250) forms the limiting channel (230), and one end of the end cap (250) has a first through hole (250a) to communicate the line placement cavity (220) with the limiting channel (230), and the other end of the end cap (250) has a second through hole (250b) to communicate the limiting channel (230) with outside of the anchoring body (200).

14. The anchor (10) according to any one of claims 1 to 6, wherein at least a part of the recovery line (400) is located in the driving tube (300) so as to enter the target tissue (40) under drive of the driving tube (300).

15. An anchoring device, **characterized by** comprising:
a puncture needle (20), wherein a distal end of the puncture needle (20) is configured to extend into a target tissue (40); and
the anchor (10) according to any one of claims 1 to 14,
wherein the anchoring body (200) of the anchor (10) moves along the puncture needle (20) under drive of the driving tube (300) to enter the target tissue (40).
